(19) Europäisches Patentamt
European Patent Office
Office européen des brevets

(11) **EP 3 984 493 B1**

(12) **EUROPÄISCHE PATENTSCHRIFT**

(45) Veröffentlichungstag und Bekanntmachung des Hinweises auf die Patenterteilung:
**04.10.2023 Patentblatt 2023/40**

(21) Anmeldenummer: **20215943.0**

(22) Anmeldetag: **21.12.2020**

(51) Internationale Patentklassifikation (IPC):
*A61C 5/73* (2017.01)    *A61C 5/77* (2017.01)
*A61C 13/00* (2006.01)    *A61C 13/08* (2006.01)
*B33Y 50/00* (2015.01)    *B33Y 80/00* (2015.01)

(52) Gemeinsame Patentklassifikation (CPC):
**A61C 5/77; A61C 5/73; A61C 13/0004;**
**A61C 13/08; A61C 13/082; A61C 13/09;**
**B33Y 50/00; B33Y 80/00**

(54) **VERFAHREN ZUM HERSTELLEN EINER DENTALEN RESTAURATION**

METHOD FOR PRODUCING A DENTAL RESTORATION

PROCÉDÉ DE RÉALISATION D'UNE RESTAURATION DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2020 EP 20201553**

(43) Veröffentlichungstag der Anmeldung:
**20.04.2022 Patentblatt 2022/16**

(73) Patentinhaber: **Ivoclar Vivadent AG**
**9494 Schaan (LI)**

(72) Erfinder:
• **ROHNER, Gottfried**
 **9450 Altstätten (CH)**
• **JOHN, Hendrik**
 **9470 Buchs (CH)**
• **JUSSEL, Rudolf**
 **6805 Feldkirch-Gisingen (AT)**
• **NIEDRIG, Christian**
 **9464 Rüthi (CH)**

(74) Vertreter: **Baldus, Oliver**
**Splanemann**
**Rumfordstrasse 7**
**80469 München (DE)**

(56) Entgegenhaltungen:
**EP-A1- 2 486 892    WO-A1-2019/023461**

EP 3 984 493 B1

## Beschreibung

Die vorliegende Erfindung betrifft ein Verfahren zum Herstellen einer dentalen Restauration, eine Computervorrichtung zum Herstellen einer dentalen Restauration und ein Computerprogramm.

Dentale Restaurationen können aus einer Vielzahl unterschiedlicher Materialien aufgebaut sein, um das Erscheinungsbild eines natürlichen Zahns möglichst naturgetreu wiederzugeben. Die Auswahl der passenden Materialien ist jedoch aufwändig und kann zu suboptimalen Ergebnissen führen. Oft ist es unklar, wie die optischen Eigenschaften der dentalen Restauration durch die jeweiligen Restaurationsmaterialien erreicht werden. Daher ist es schwierig, eine neue dentale Restauration mit den gewünschten optischen Eigenschaften herzustellen.

Die Druckschrift WO2019/023461 A1 betrifft ein Verfahren zum Herstellen einer Zahnrestauration für einen Patienten, sodass die Zahnrestauration optische Eigenschaften aufweist, die den optischen Eigenschaften der Zähne des Patienten entsprechen. Hierzu wird ein Zwischenmodel (Intermediate Model) mit Startwerten für die optischen Eigenschaften (Starting Optical Property Values) gebildet.

Es ist die technische Aufgabe der vorliegenden Erfindung, eine Materialkombination für eine dentale Restauration zu bestimmen, dass diese einem gewünschten natürlichen Erscheinungsbild entspricht.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Herstellen einer dentalen Restauration gelöst, mit den Schritten eines Renderns eines ersten digitalen Zahnmodells mit einer ersten Materialkombination zum Erzeugen eines ersten Ist-Datensatzes, der die optischen Eigenschaften des ersten digitalen Zahnmodells wiedergibt; eines Bestimmens einer ersten Abweichung zwischen einem Soll-Datensatz und dem ersten Ist-Datensatz; eines Renderns eines zweiten digitalen Zahnmodells mit einer zweiten Materialkombination zum Erzeugen eines zweiten Ist-Datensatzes, der die optischen Eigenschaften des zweiten digitalen Zahnmodells wiedergibt; eines Bestimmens einer zweiten Abweichung zwischen dem Soll-Datensatz und dem zweiten Ist-Datensatz; und eines Herstellens der dentalen Restauration auf Basis des ersten digitalen Zahnmodells, wenn die erste Abweichung kleiner als die zweite Abweichung ist und eines Herstellens der dentalen Restauration auf Basis des zweiten digitalen Zahnmodells, wenn die zweite Abweichung kleiner als die erste Abweichung ist.

Durch das Verfahren kann für eine herzustellende dentale Restauration durch eine Variation der Zuordnung verschiedener Restaurationsmaterialien zu einer inneren Architektur des Zahnmodells im Voraus berechnet werden, welches Erscheinungsbild die spätere dentale Restauration aufweist. Aus einer Vielzahl an Kombinationen kann das optimale Ergebnis ermittelt werden, indem die optischen Erscheinungsbilder der jeweiligen Ist-Datensätze mit dem festgelegten Soll-Datensatz verglichen werden. Der Soll-Datensatz kann zuvor auf Basis eines Nachbarzahn ermittelt worden sein. Auf diese Weise kann eine Materialkombination und -Zuordnung für die Herstellung der dentalen Restauration festgelegt werden. Die äußere Form und innere Architektur der dentalen Restauration können dabei vorgegeben werden.

In einer technisch vorteilhaften Ausführungsform des Verfahrens gibt das erste digitale Zahnmodell und das zweite digitale Zahnmodell die gleiche räumliche Geometrie wieder.

Dadurch wird beispielsweise der technische Vorteil erreicht, dass das Verfahren mit einer höheren Genauigkeit durchgeführt werden kann.

In einer weiteren technisch vorteilhaften Ausführungsform des Verfahrens wird das Rendern des ersten digitalen Zahnmodells mittels eines ersten Prozessors und das Rendern des zweiten digitalen Zahnmodells mittels eines zweiten Prozessors durchgeführt wird. Dadurch wird beispielsweise der technische Vorteil erreicht, dass die Rendering-Schritte zeitgleich ausgeführt werden können und das Verfahren schneller durchgeführt werden kann.

In einer weiteren technisch vorteilhaften Ausführungsform des Verfahrens wird das Rendern des ersten digitalen Zahnmodells parallel zu einem Rendern des zweiten digitalen Zahnmodells durchgeführt wird. Dadurch wird beispielsweise ebenfalls der technische Vorteil erreicht, dass die Rendering-Schritte zeitglich ausgeführt werden können und das Verfahren schneller durchgeführt werden kann.

In einer weiteren technisch vorteilhaften Ausführungsform des Verfahrens wird die erste und/oder zweite Abweichung auf Basis eines euklidischen Abstandes zwischen dem Soll-Datensatz und dem Ist-Datensatz oder auf Basis eines spektralen Abstandes zwischen dem Soll-Datensatz und dem Ist-Datensatz berechnet. Dadurch wird beispielsweise ebenfalls der technische Vorteil erreicht, dass die Abweichung mit einer hohen Genauigkeit berechnet werden kann.

In einer weiteren technisch vorteilhaften Ausführungsform des Verfahrens umfasst die erste und/oder die zweite Materialkombination zumindest zwei unterschiedliche Restaurationsmaterialien. Dadurch wird beispielsweise ebenfalls der technische Vorteil erreicht, dass eine dentale Restauration mit einem möglichst natürlichen Erscheinungsbild erhalten wird.

In einer weiteren technisch vorteilhaften Ausführungsform des Verfahrens ist der räumliche Aufbau des ersten und/oder des zweiten digitalen Zahnmodells vorgegeben. Dadurch wird beispielsweise der technische Vorteil erreicht, dass der Aufbau der digitalen Zahnmodelle konstant bleibt und nur die Materialkombinationen variiert werden, was die Berechnungszeit des Verfahrens verkürzt.

In einer weiteren technisch vorteilhaften Ausführungsform des Verfahrens wird das Rendern auf Basis von Farbwerten, Reflexions-, Transmissions- und/oder Absorptionswerten der jeweiligen Restaurationsmaterialien durchgeführt. Dadurch wird beispielsweise der technische Vorteil erreicht, dass ein hochgenaues Rendering durchgeführt wird, dass das spätere Aussehen der dentalen Restauration naturgetreu wiedergibt.

In einer weiteren technisch vorteilhaften Ausführungsform des Verfahrens werden beim Rendern die optischen Eigenschaften einer Zementschicht, Komposit-Schicht, Adhäsiv-Schicht und/oder der Farbeindruck der Präparation zusätzlich berücksichtigt. Dadurch wird beispielsweise der technische Vorteil erreicht, dass ein naturgetreueres Aussehen beim Rendern erzielt wird und sowohl Materialien für das spätere Befestigen der dentalen Restauration als auch die Präparation berücksichtigt werden.

In einer weiteren technisch vorteilhaften Ausführungsform des Verfahrens wird eine bestimmte Anzahl von Restaurationsmaterialien vorgegeben und das Verfahren für alle möglichen Materialkombinationen wiederholt. Dabei kann jeder Rendering-Schritt parallel durchgeführt werden. Dadurch wird beispielsweise der technische Vorteil erreicht, dass sich die dentale Restauration möglichst präzise anpassen lässt.

In einer weiteren technisch vorteilhaften Ausführungsform des Verfahrens wird diejenige Materialkombination zum Herstellen ausgewählt, die die geringste Abweichung zwischen dem Soll-Datensatz und dem jeweiligen Ist-Datensatz aufweist. Dadurch wird beispielsweise der technische Vorteil erreicht, dass bei gegebenen Restaurationsmaterialien das optimale Ergebnis erreicht wird.

In einer weiteren technisch vorteilhaften Ausführungsform des Verfahrens weist das digitale Zahnmodells eine vorgegebene äußere Form und eine vorgegebene innere Struktur auf. Dadurch wird beispielsweise der technische Vorteil erreicht, dass die Berechnungsgrundlage verbessert wird.

In einer weiteren technisch vorteilhaften Ausführungsform des Verfahrens wird der Soll-Datensatz auf Basis eines natürlichen Zahns erhalten. Dadurch wird beispielsweise der technische Vorteil erreicht, dass die dentale Restauration an einen natürlichen Zahn angepasst werden kann.

In einer weiteren technisch vorteilhaften Ausführungsform des Verfahrens gibt der Soll-Datensatz die optischen Eigenschaften und/oder die Geometrie des natürlichen Zahns wieder. Dadurch wird beispielsweise der technische Vorteil erreicht, dass die Wiedergabetreue der dentalen Restauration nochmals verbessert wird.

Gemäß einem zweiten Aspekt wird die technische Aufgabe durch eine Computervorrichtung zum Herstellen einer dentalen Restauration gelöst, mit einer Herstellungsvorrichtung, die geeignet ist, das Verfahren nach dem ersten Aspekt auszuführen. Dadurch werden die gleichen technischen Vorteile wie durch das Verfahren nach dem ersten Aspekt erreicht.

Gemäß einem dritten Aspekt wird die technische Aufgabe durch ein Computerprogramm gelöst, umfassend Befehle, die bewirken, dass die Computervorrichtung nach dem zweiten Aspekt die Verfahrensschritte nach dem ersten Aspekt ausführt. Dadurch werden die gleichen technischen Vorteile wie durch das Verfahren nach dem ersten Aspekt erreicht.

Ausführungsbeispiele der Erfindung sind in den Zeichnungen dargestellt und werden im Folgenden näher beschrieben.

Es zeigen:

Fig. 1    eine schematische Darstellung digitaler Zahnmodelle;

Fig. 2    eine Darstellung eines Vergleichs zwischen einem Soll-Datensatz und einem Ist-Datensatz;

Fig. 3    eine schematische Darstellung zur Berechnung einer Abweichung zwischen einem Soll-Datensatz und einem Ist Datensatz;

Fig. 4    ein Blockdiagramm eines Verfahrens zum Herstellen einer dentalen Restauration.

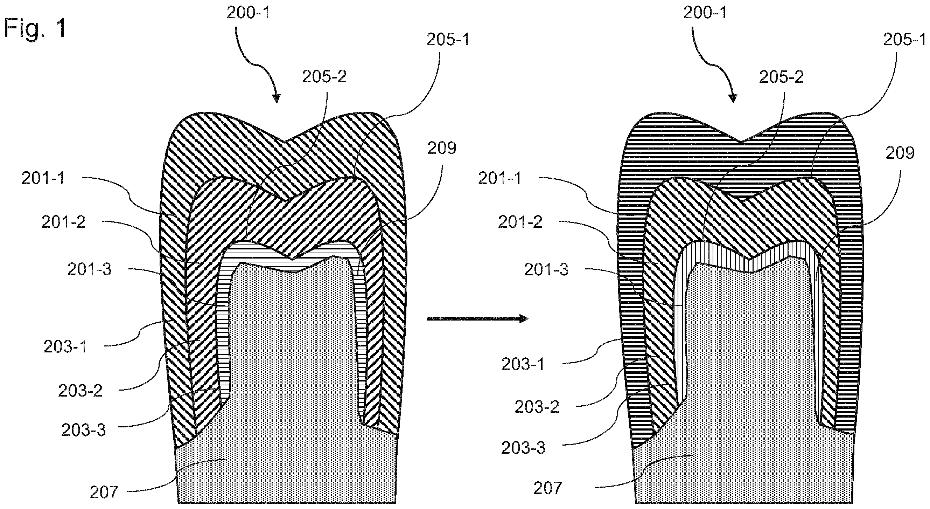

Fig. 1 zeigt eine schematische Darstellung digitaler Zahnmodelle 200-1 und 200-2 für eine dentale Restauration 100.

Durch die digitalen Zahnmodelle 200-1 und 200-2 wird die äußere Form und der innere räumliche Aufbau der späteren dentalen Restauration 100 vorgegeben. Die äußere Form kann hierzu in einem CAD-Programm erzeugt werden. Der innere räumliche Aufbau kann errechnet oder aus Datenbanken entnommen werden, beispielsweise abgeleitet vom Aufbau eines natürlichen Zahns oder aus der äußeren Form der dentalen Restauration 100 oder eines natürlichen Zahns errechnet werden. Bei vorgegebener äußerer Form der dentalen Restauration 100 kann durch die Zahnmodelle 200-1 und 200-2 ein innerer Schichtaufbau festgelegt werden und jeder einzelnen Schicht ein eigenes Restaurationsmaterial 201 und 203 mit vorgegebenen optischen Materialparametern zugewiesen werden. Für die Zahnmodelle wird das Erscheinungsbild desselben Restzahns 207, bzw. derselben Präparation des zu behandelnden Zahns angenommen.

Das erste Zahnmodell 200-1 gibt beispielsweise eine dentale Restauration 100 wieder, die schichtweise aus den unterschiedlichen Restaurationsmaterialen 201-1, 201-2 und 201-3 aufgebaut ist. In einer äußeren Schicht wird das Restaurationsmaterial 201-1 verwendet, in einer mittleren Schicht wird das Restaurationsmaterial 201-2 verwendet und in einer inneren Schicht wird das Restaurationsmaterial 201-3 verwendet. Die innere Schicht kann auch eine Befestigungsschicht 209 sein.

Das zweite Zahnmodell 200-2 gibt die gleiche dentale Restauration 100 wieder, die ebenfalls schichtweise aus unterschiedlichen Restaurationsmaterialen 203-1, 203-2 und 203-3 aufgebaut ist. Allerdings wird in einer äußeren Schicht das Restaurationsmaterial 203-1 verwendet, in einer mittleren Schicht wird das Restaurationsmaterial 203-2 verwendet und in einer inneren Schicht wird das Restaurationsmaterial 203-3 verwendet. Die innere Schicht kann auch eine Befestigungsschicht 209 mit einem Befestigungsmaterial 203-3 sein. Die Materialkombination des ersten Zahnmodells 200-1 unterscheidet sich daher von der Materialkombination des zweiten Zahnmodels 200-2.

Die optischen und physikalischen Eigenschaften der jeweils zugewiesenen Restaurationsmaterialien 201-1, ..., 201-3, 203-1, ..., und 203-3 sind bekannt, wie beispielsweise Farbwerte, Streuwerte, Reflexionswerte, Transmissionswerte und/oder Absorptionswerte.

Durch ein Rendering (Lichtsimulationsverfahren) mittels Strahlverfolgung (Ray Tracing) können die Farbe und die Transluzenz der dentalen Restauration auf Basis der zuvor erstellten Zahnmodelle 200 berechnet werden. Auf diese Weise kann aus den Zahnmodellen 200 das spätere Aussehen und der optische Eindruck des biomimetischen Zahnersatzes errechnet werden, wie beispielsweise einer Brücke, Krone, Teilkrone, Inlay, Onlay oder einem Veneer.

Das Rendern wird mittels einer physikalisch korrekten Simulation der Wechselwirkung von Licht und der geplanten dentalen Restauration 100 und den verwendeten Restaurationsmaterialien durchgeführt. Dabei werden die bekannten optischen Parameter der einzelnen Restaurationsmaterialien verwendet, um eine computergestützte Ansicht der dentalen Restauration 100 zu erzeugen.

Hierzu kann zusätzlich bestehendes natürliches Zahnmaterial berücksichtigt werden, wie beispielsweise ein Restzahn, auf dem die dentale Restauration 100 aufgesetzt werden soll. Beim Rendern wird zu dem vorgegebenen inneren Aufbau und den ausgewählten Restaurationsmaterialien der optische Eindruck der späteren dentalen Restauration 100 errechnet. Für das Rendern kann computergestützt eine Berechnung von Reflexions-, Transmissions- und Absorptionswerten im sichtbaren Bereich bei mind. drei Wellenlängen durchgeführt werden.

Für das Verfahren zur Herstellung wird zunächst ein Soll-Datensatz ermittelt. Der Soll-Datensatz kann durch ein optisches Erfassen und Auswerten eines benachbarten Zahns erhalten werden. Hierzu kann eine elektronische Kamera oder ein 3D-Scanner verwendet werden, mit dem die Farbwerte, Reflexions-, Transmissions- und/oder Absorptionswerte und die räumliche Form oder ein Bild des natürlichen Zahns bestimmt werden können. Auf Basis dieser Daten wird die dentale Restauration 100 mit möglichst identischen Eigenschaften geplant und ein räumliches Zahnmodell 200 geplant.

Der Soll-Datensatz dient dazu das gerenderte Erscheinungsbild der dentalen Restauration 100 unter Variation Restaurationsmaterialien, die der inneren Architektur zugeordnet sind, solange zu vergleichen, bis eine optimale Annäherung an den Soll-Datensatz gefunden wird.

Idealerweise wird für den Vergleich das Rendern der dentalen Restauration 100 aus demselben Blickwinkel oder derselben Perspektive durchgeführt, aus der der Soll-Datensatz auf Basis des natürlichen Zahns gewonnen worden ist. Zur Verbesserung der Ergebnisse kann das Rendern auch aus verschiedenen Blickwinkeln erfolgen. Das Rendern kann für jeden beliebigen Blickwinkel und beliebig wählbare Umgebungssituationen erfolgen, wie beispielsweise einer vorgegebenen Beleuchtungssituation, unter Berücksichtigung von Nachbarzähnen, einer Position der dentalen Restauration 100 im Mundraum oder einer Form und optischen Eigenschaften des zu präparierenden Zahnrestes. Beim Rendern können auch weitere beeinflussende Bedingungen berücksichtigt werden, wie beispielsweise bekannte optische Daten einer Befestigungsschicht 209 (Zement-, Komposit- und/oder Adhäsiv).

Durch das Rendern der digitalen Zahnmodelle 200-1 und 200-2 wird somit ein Ist-Datensatzes erhalten, der die optischen Eigenschaften der digitalen Zahnmodelle 200-1 und 200-2 wiedergibt.

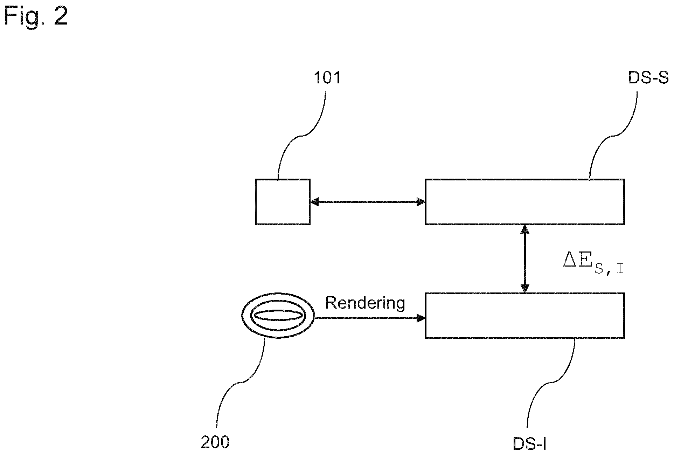

Fig. 2 zeigt eine schematische Darstellung einer Abweichung $\Delta E_{S,I}$ zwischen dem Soll-Datensatz DS-S und dem Ist-Datensatz DS-I (DS-I-1, DS-I-2). Der Soll-Datensatz DS-S wird beispielsweise durch eine elektronische Kamera 101 erhalten.

Das digital erzeugte Zahnmodell 200 umfasst Daten über die räumliche Geometrie der dentalen Restauration 100 und die zugeordneten Restaurationsmaterialien, aus denen die dentale Restauration 100 hergestellt werden soll und die Bereiche, in denen die Restaurationsmaterialien angeordnet werden sollen. Die optischen und physikalischen Eigenschaften der Restaurationsmaterialien, die für das Rendern erforderlich sind, sind in der Rendering-Software bekannt. Diese können aus einer Parametertabelle entnommen werden, die ständig um neue Materialien ergänzt wird.

Der Ist-Datensatz DS-I wird erhalten, indem das digitale Zahnmodell 200 mit einer ausgewählten Materialkombination einem Rendering unterzogen wird. Beim Rendering werden die räumliche Geometrie der dentalen Restauration 100 und die optischen und physikalischen Eigenschaften der verschiedenen, vorkommenden Restaurationsmaterialien, ggf. einschließlich Befestigungsmaterial und Restzahn bzw. Präparation berücksichtigt.

Die Ausbreitung von Licht in dem Zahnmodell 200 kann durch die Maxwell-Gleichungen beschrieben werden. Das Rendering verwendet beispielsweise die Strahlungstransportgleichung (RTE - Radiative Transport Equation), bei der ein Ausbreitungsmedium durch den Absorptionskoeffizienten, den Streukoeffizienten, den Brechungsindex und die Streuphasenfunktion beschrieben wird.

Das digitale Zahnmodell 200 umfasst die Daten über die räumliche Geometrie der dentalen Restauration 100 und die innere Architektur sowie den Absorptionskoeffizienten, den Streukoeffizienten, den Brechungsindex und die Streuphasenfunktion für die jeweiligen Restaurationsmaterialien 201 und 203 der Architektur. Die Streuphasenfunktion kann beim Rendern auf Basis des Zahnmodells 200 mit den genannten Parametern in einer Monte-Carlo-Simulation in jeder gewünschten Genauigkeit numerisch gelöst werden, bei der eine Vielzahl von Photonen auf zufälligen Pfaden durch das Zahnmodell 200 propagieren.

Daraus lässt sich durch das Rendern ein Ist-Datensatz DS-I für das Aussehen der dentalen Restauration 100 errechnen, der die verwendeten Materialien, die äußere Form und die innere Architektur des Zahnmodells 200 berücksichtigt. Dieser berechnete Ist-Datensatz DS-I kann anschließend mit dem Soll-Datensatz DS-S, der auf Basis eines Nachbarzahns gewonnen worden ist, verglichen werden. Zur Vereinfachung des Vergleichs kann dieser in einer zweidimensionalen Ableitung (zweidimensionales Bild) erfolgen. Im Allgemeinen können jedoch auch dreidimensionale Verfahren verwendet werden. Als Maß für eine Abweichung $\Delta E_{S,I}$ zwischen dem Ist-Datensatz DS-I und dem Soll-Datensatz DS-S wird ein numerischer Wert berechnet.

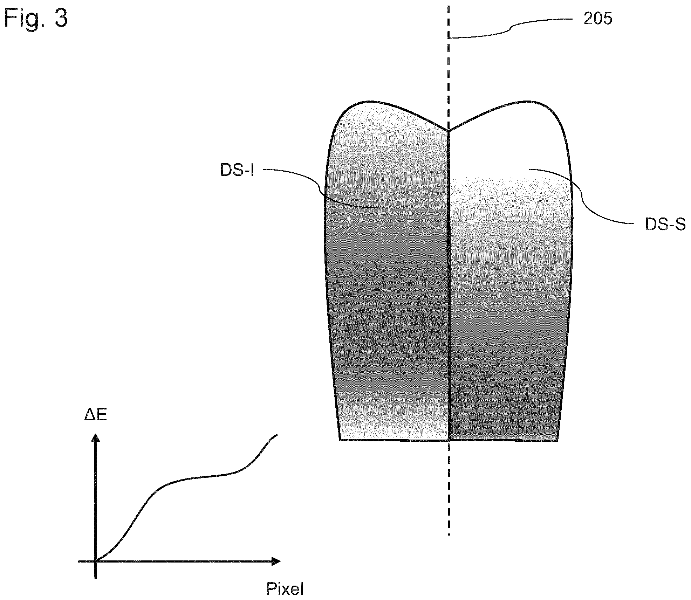

Fig. 3 zeigt eine schematische Darstellung zur Berechnung einer Abweichung $\Delta E_{S,I}$ zwischen dem Soll-Datensatz DS-S und einem Ist-Datensatz DS-I. Beispielsweise umfasst der Datensatz DS-S eine Darstellung des Zahns in einer vorgegebenen Perspektive. Demgegenüber umfasst der Datensatz DS-I, der aus dem Zahnmodell 200 gerendert worden ist, eine Darstellung des Zahnmodells 200 in der gleichen Perspektive. Die beiden Abbildungen aus dem Soll-Datensatz DS-S und dem Ist-Datensatz DS-I werden auf die gleiche Größe skaliert und nebeneinandergesetzt.

Die euklidische Abweichung $\Delta E_{S,I}$ ($\Delta E1_{S,I}$, $\Delta E2_{S,I}$) zwischen dem Soll-Datensatz DS-S und dem Ist-Datensatz DS-I kann berechnet werden, indem die Unterschiede in den Farbwerten der Pixel entlang der Vergleichslinien 205 aufsummiert werden, beispielsweise im L*a*b*-Farbraum. Diese Vergleichslinien 205 können beliebig verschoben werden, indem beispielsweise die Zahnhälften zusammengeschoben werden. Im Allgemeinen kann die Vergleichslinie 205 jedoch auch einen anderen Verlauf aufweisen. Der Vergleich kann auf Pixel-Ebene als kleinste Auflösung erfolgen.

$$\Delta E_{S,I} = \sqrt{(L_S^* - L_I^*)^2 + (a_S^* - a_I^*)^2 + (b_S^* - b_I^*)^2}$$

Je größer der Unterschied im Farbverlauf entlang der Vergleichslinie 205 ist, desto größer ist die numerische Abweichung $\Delta E_{S,I}$. Bei vollkommener farblicher Übereinstimmung zwischen dem Soll-Datensatz DS-S und dem Ist-Datensatz DS-I ist die Abweichung $\Delta E_{S,I}$ Null. Im Allgemeinen können jedoch auch andere Verfahren zum Berechnen der Abweichung $\Delta E_{S,I}$ herangezogen werden, wie beispielsweise auf der Basis von Spektralinformation.

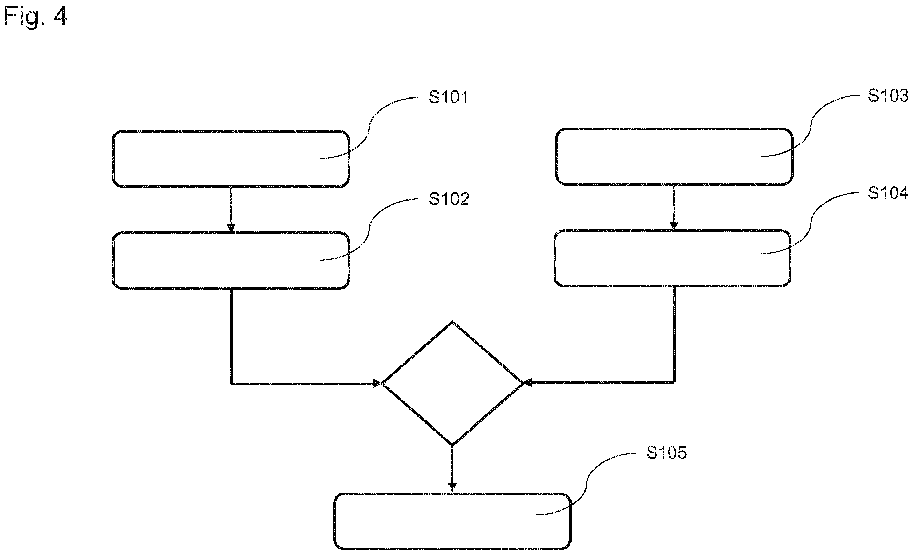

Fig. 4 zeigt ein Blockdiagramm des Verfahrens zum Herstellen der dentalen Restauration 100. In ersten Schritt S101 wird das erste digitale Zahnmodell 200-1 mit einer ersten Materialkombination zum Erzeugen eines ersten Ist-Datensatzes DS-I-1 gerendert, der die optischen Eigenschaften des ersten digitalen Zahnmodells 200-1 wiedergibt. Im Schritt S102 wird dieser erste Ist-Datensatz DS-I-1 mit dem Soll-Datensatz DS-S verglichen, um eine erste Abweichung $\Delta E1_{S,I}$ zwischen dem Soll-Datensatz DS-S und dem ersten Ist-Datensatz DS-I-1 zu erhalten.

Im Schritt S103 wird dann das zweite digitale Zahnmodell 200-2 mit einer zweiten Materialkombination zum Erzeugen eines zweiten Ist-Datensatzes DS-I-2 gerendert, der die optischen Eigenschaften des zweiten digitalen Zahnmodells 200-2 wiedergibt. Im Schritt S104 wird dieser zweite Ist-Datensatz DS-I-2 ebenfalls mit dem Soll-Datensatz DS-S verglichen, um eine zweite Abweichung $\Delta E2_{S,I}$ zwischen dem Soll-Datensatz DS-S und dem zweiten Ist-Datensatz DS-I-1 zu erhalten.

Im Schritt S105 wird dann die dentale Restauration 100 auf Basis des ersten digitalen Zahnmodells 200-1 hergestellt, wenn die erste Abweichung $\Delta E1_{S,I}$ kleiner als die zweite Abweichung $\Delta E2_{S,I}$ ist und oder auf Basis des zweiten digitalen Zahnmodells 200-1 hergestellt, wenn die zweite Abweichung $\Delta E2_{S,I}$ kleiner als die erste Abweichung $\Delta E1_{S,I}$ ist .

Mit dem Verfahren können alle infrage kommenden Materialkombinationen des geometrisch gegebenen Gesamtsystems gerendert werden. Werden alle möglichen Materialkombinationen berechnet, lässt sich beispielsweise eine beste Kombination ermitteln, die dem Soll-Datensatz DS-S an nächsten kommt und durch die das natürliche Aussehen des Zahns optimal nachgeahmt werden kann. Durch das Verfahren wird ein objektiver Soll-IstVergleich erhalten und durch Computerunterstützung eine optimale Zuordnung von Restaurationsmaterialien zu den jeweiligen räumlichen Bereichen der dentalen Restauration 100 erreicht (Best Match).

Bei gleichbleibendem innerem Aufbau der Zahnmodelle 200-1 und 200-2 werden hierzu eine Veränderung der gewählten Materialkombination für einzelne Schichten berechnet. Anschließend wird die beste Kombination ermittelt.

Das Rendern der digitale Zahnmodelle 200-1 und 200-2 kann parallel oder zeitgleich auf unterschiedlichen Prozessoren durchgeführt werden, so dass in kurzer Zeit optimale Ergebnisse erhalten werden. Durch das parallele Rendern verschiedener möglicher Variationen der Materialzuordnung wird das schnelle Finden einer optisch patientenspezifisch passenden dentalen Restauration 100 gelöst. Eine iterative Berechnung ist in diesem Fall nicht nötig.

Eine parallele Berechnung und Simulation aller möglichen Materialkombinationen für ein Zahnmodell 200 mit vorgegebener äußerer Form und innerer Architektur und einem anschließenden Vergleich der erhaltenen Ist-Datensätze mit dem Soll-Datensatz kann zur Ermittlung einer bestmöglichen Materialkombination verwendet werden.

Zudem ist es in dem Verfahren möglich, Zahnmodelle 200-1 und 200-2 mit virtuellen Restaurationsmaterialien zu berechnen, denen vorgegebene optische Eigenschaften zuvor zugewiesen worden sind. Auch hieraus kann eine geeignete Materialkombination ermittelt werden. Anschließend kann die dentale Restauration 100 mit realen Restaurationsmaterialien hergestellt werden, deren Eigenschaften den virtuellen Restaurationsmaterialien 201 und 203 nahekommen. Auch auf diese Weise kann der gewünschte Gesamteindruck der Restauration 100 erzielt werden.

Durch das Verfahren kann eine optimale Auswahl und Zuordnung von Restaurationsmaterialien 201 und 203 zur Erzeugung der mehrschichtigen Restauration 100 vor der Fertigung der Restauration 100 bestimmt und gleichzeitig eine optimale Ästhetik der Restauration 100 sichergestellt werden. Dadurch, dass mit den physikalischen und optischen Parametern existierender Restaurationsmaterialien gerendert wird, kann anschließend eine dentale Restauration 100 mit einer optimale Materialkombination hergestellt werden.

Sobald die Materialkombination ermittelt worden ist, kann die dentale Restauration 100 durch ein 3D-Druckverfahren oder ein anderes geeignetes Verfahren mit den jeweiligen Restaurationsmaterialien hergestellt werden. In diesem Fall kann eine Computervorrichtung verwendet werden, die die Berechnungsschritte ausführt und anschließend die dentale Restauration 100 mittels der Herstellungsvorrichtung herstellt. Die Computervorrichtung führt zu diesem Zweck ein Computerprogramm aus, das Befehle umfasst, die bewirken, dass die Computervorrichtung die erforderlichen Verfahrensschritte ausführt. Die Computervorrichtung umfasst einen Prozessor und einen digitalen Speicher, in dem die Datensätze und ein Computerprogramm gespeichert ist, das die Verfahrensschritte ausführt und eine Herstellungsvorrichtung geeignet ansteuert. Die Herstellungsvorrichtung ist beispielsweise ein 3D-Drucker, der die dentale Restauration 100 mit den unterschiedlichen Restaurationsmaterialien druckt. Im Allgemeinen können jedoch auch andere Herstellungsvorrichtungen verwendet werden, die die dentale Restauration mit den unterschiedlichen Restaurationsmaterialien herstellen kann.

BEZUGSZEICHENLISTE

| | |
|---|---|
| 100 | Dentale Restauration |
| 101 | elektronische Kamera |
| 200 | Zahnmodell |
| 201 | Restaurationsmaterial |
| 203 | Restaurationsmaterial |
| 205 | Vergleichslinie |
| 207 | Restzahn |
| 209 | Befestigungsschicht |

| | |
|---|---|
| DS-I | Ist-Datensatz |
| DS-S | Soll- Datensatz |

**Patentansprüche**

1. Verfahren zum Herstellen einer dentalen Restauration (100), mit den Schritten:

- Rendern (S101) eines ersten digitalen Zahnmodells (200-1) mit einer ersten Materialkombination zum Erzeugen eines ersten Ist-Datensatzes (DS-I-1), der die optischen Eigenschaften des ersten digitalen Zahnmodells (200-1) wiedergibt;
- Bestimmen (S102) einer ersten Abweichung ($\Delta E1_{S,I}$) zwischen einem Soll-Datensatz (DS-S) und dem ersten Ist-Datensatz (DS-I-1);
- Rendern (S103) eines zweiten digitalen Zahnmodells (200-2) mit einer zweiten Materialkombination zum Erzeugen eines zweiten Ist-Datensatzes (DS-I-2), der die optischen Eigenschaften des zweiten digitalen Zahnmodells (200-2) wiedergibt;

- Bestimmen (S104) einer zweiten Abweichung ($\Delta E2_{S,I}$) zwischen dem Soll-Datensatz (DS-S) und dem zweiten Ist-Datensatz (DS-I-2); und **gekennzeichnet durch** den weiteren Schritt:
- Herstellen (S105) der dentalen Restauration (100) auf Basis des ersten digitalen Zahnmodells (200-1), wenn die erste Abweichung ($\Delta E1_{S,I}$) kleiner als die zweite Abweichung ($\Delta E2_{S,I}$) ist und Herstellen der dentalen Restauration (100) auf Basis des zweiten digitalen Zahnmodells (200-1), wenn die zweite Abweichung ($\Delta E2_{S,I}$) kleiner als die erste Abweichung ($\Delta E1_{S,I}$) ist.

2. Verfahren nach Anspruch 1, wobei das erste digitale Zahnmodell (200-1) und das zweite digitale Zahnmodell (200-1) die gleiche räumliche Geometrie wiedergeben.

3. Verfahren nach einem der vorangehenden Ansprüche, wobei das Rendern (S101) des ersten digitalen Zahnmodells (200-1) mit ersten Materialkombination mittels eines ersten Prozessors und das Rendern (S103) des zweiten digitalen Zahnmodells (200-2) mit der zweiten Materialkombination mittels eines zweiten Prozessors durchgeführt wird.

4. Verfahren nach einem der vorangehenden Ansprüche, wobei die erste und/oder zweite Abweichung ($\Delta E1_{S,I}$, $\Delta E2_{S,I}$) auf Basis eines euklidischen Abstandes zwischen dem Soll-Datensatz (DS-S) und dem Ist-Datensatz (DS-I) oder auf Basis eines spektralen Abstandes zwischen dem Soll-Datensatz (DS-S) und dem Ist-Datensatz (DS-I) berechnet wird.

5. Verfahren nach einem der vorangehenden Ansprüche, wobei die ersten und/oder die zweite Materialkombination zumindest zwei unterschiedliche Restaurationsmaterialien (201-1, ..., 203-1, ..., 203-1, ..., 203-3) umfasst.

6. Verfahren nach einem der vorangehenden Ansprüche, wobei der räumliche Aufbau des ersten und/oder des zweiten digitalen Zahnmodells (200-1, 200-2) vorgegeben ist.

7. Verfahren nach einem der vorangehenden Ansprüche, wobei das Rendern (S101, S103) auf Basis von Farbwerten, Reflexions-, Transmissions- und/oder Absorptionswerten der jeweiligen Restaurationsmaterialien durchgeführt wird.

8. Verfahren nach einem der vorangehenden Ansprüche, wobei das Rendern (S101, S103) beim Rendern die optischen Eigenschaften einer Zementschicht, Komposit-Schicht, Adhäsiv-Schicht und/oder der Farbeindruck der Präparation zusätzlich berücksichtigt wird.

9. Verfahren nach einem der vorangehenden Ansprüche, wobei eine bestimmte Anzahl von Restaurationsmaterialien vorgegeben wird und das Verfahren für alle möglichen Materialkombinationen wiederholt wird.

10. Verfahren nach Anspruch 9, wobei diejenige Materialkombination zum Herstellen ausgewählt wird, die die geringste Abweichung zwischen dem Soll-Datensatz (DS-S) und dem jeweiligen Ist-Datensatz aufweist.

11. Verfahren nach einem der vorangehenden Ansprüche, wobei das digitale Zahnmodell eine vorgegebene äußere Form und eine vorgegebene innere Struktur aufweist.

12. Verfahren nach einem der vorangehenden Ansprüche, wobei der Soll-Datensatz (DS-S) auf Basis eines natürlichen Zahns erhalten wird.

13. Verfahren nach Anspruch 12, wobei der Soll-Datensatz (DS-S) die optischen Eigenschaften und/oder die Geometrie des natürlichen Zahns wiedergibt.

14. Computervorrichtung zum Herstellen einer dentalen Restauration (100), mit einer Herstellungsvorrichtung, die geeignet ist, das Verfahren nach einem der Ansprüche 1 bis 13 auszuführen.

15. Computerprogramm, umfassend Befehle, die bewirken, dass die Computervorrichtung des Anspruchs 14 die Verfahrensschritte nach einem der Ansprüche 1 bis 13 ausführt.

**Claims**

1. A method for producing a dental restoration (100), comprising the steps of:

- rendering (S101) a first digital tooth model (200-1) with a first material combination to generate a first actual data set (DS-I-1) representing the optical properties of the first digital tooth model (200-1);
- determining (S102) a first deviation ($\Delta E1_{S,I}$) between a target data set (DS-S) and the first actual data set (DS-I-1) ;
- rendering (S103) a second digital tooth model (200-2) with a second material combination to generate a second actual data set (DS-I-2) representing the optical properties of the second digital tooth model (200-2);
- determining (S104) a second deviation ($\Delta E2_{S,I}$) between the target data set (DS-S) and the second actual data set (DS-I-2); and **characterized by** the further step of:
- producing (S105) the dental restoration (100) based on the first digital tooth model (200-1) if the first deviation ($\Delta E1_{S,I}$) is smaller than the second deviation ($\Delta E2_{S,I}$), and producing the dental restoration (100) based on the second digital tooth model (200-2) if the second deviation ($\Delta E2_{S,I}$) is smaller than the first deviation ($\Delta E1_{S,I}$).

2. The method according to claim 1, wherein the first digital tooth model (200-1) and the second digital tooth model (200-2) represent the same spatial geometry.

3. The method according to any one of the preceding claims, wherein the rendering (S101) of the first digital tooth model (200-1) with the first material combination is performed by means of a first processor and the rendering (S103) of the second digital tooth model (200-2) with the second material combination is performed by means of a second processor.

4. The method according to any one of the preceding claims, wherein the first and/or second deviation ($\Delta E1_{S,I}$, $\Delta E2_{S,I}$) is calculated on the basis of a Euclidean distance between the target data set (DS-S) and the actual data set (DS-I) or on the basis of a spectral distance between the target data set (DS-S) and the actual data set (DS-I).

5. The method according to any one of the preceding claims, wherein the first and/or the second material combination comprises at least two different restoration materials (201-1, ..., 203-1, ..., 203-1, ..., 203-3) .

6. The method according to any one of the preceding claims, wherein the spatial structure of the first and/or the second digital tooth model (200-1, 200-2) is predetermined.

7. The method according to any one of the preceding claims, wherein the rendering (S101, S103) is performed based on color values, reflectance values, transmission values and/or absorbance values of the respective restoration materials.

8. The method according to any one of the preceding claims, wherein the rendering (S101, S103) additionally takes into account the optical properties of a cement layer, composite layer, adhesive layer and/or the color impression of the preparation during rendering.

9. The method according to any one of the preceding claims, wherein a specific number of restoration materials is predetermined and the method is repeated for all possible material combinations.

10. The method according to claim 9, wherein the material combination selected for production is the one which has the smallest deviation between the target data set (DS-S) and the respective actual data set.

11. The method according to any one of the preceding claims, wherein the digital tooth model has a predetermined outer shape and a predetermined inner structure.

12. The method according to any one of the preceding claims, wherein the target data set (DS-S) is obtained on the basis of a natural tooth.

13. The method according to claim 12, wherein the target data set (DS-S) represents the optical properties and/or the geometry of the natural tooth.

14. A computer device for producing a dental restoration (100), comprising a production device adapted to perform the method of any one of claims 1 to 13.

15. A computer program comprising instructions that cause the computer device of claim 14 to perform the method steps of any one of claims 1 to 13.

**Revendications**

1. Procédé de fabrication d'une restauration dentaire (100), comprenant les étapes consistant à :

   - rendre (S101) un premier modèle dentaire numérique (200-1) avec une première combinaison de matériaux pour générer un premier ensemble de données réelles (DS-I-1) qui reproduit les propriétés optiques du premier modèle dentaire numérique (200-1) ;
   - déterminer (S102) un premier écart ($\Delta E1_{S,I}$) entre un jeu de données de consigne (DS-S) et le premier jeu de données réel (DS-I-1) ;
   - rendre (S103) un deuxième modèle dentaire numérique (200-2) avec une deuxième combinaison de matériaux pour produire un deuxième ensemble de données réelles (DS-I-2) qui reflète les propriétés optiques du deuxième modèle dentaire numérique (200-2) ;
   - déterminer (S104) un deuxième écart ($\Delta E2_{S,I}$) entre l'ensemble de données cible (DS-S) et le deuxième ensemble de données réel (DS-I-2) ; et **caractérisé par** l'étape supplémentaire :
   - fabriquer (S105) la restauration dentaire (100) sur la base du premier modèle dentaire numérique (200-1) lorsque le premier écart ($\Delta E1_{S,I}$) est inférieur au deuxième écart ($\Delta E2_{S,I}$) et fabriquer la restauration dentaire (100) sur la base du deuxième modèle dentaire numérique (200-1) lorsque le deuxième écart ($\Delta E2_{S,I}$) est inférieur au premier écart ($\Delta E1_{S,I}$).

2. Procédé selon la revendication 1, où le premier modèle dentaire numérique (200-1) et le deuxième modèle dentaire numérique (200-2) reproduisent la même géométrie spatiale.

3. Procédé selon l'une des revendications précédentes, où le rendu (S101) du premier modèle dentaire numérique (200-1) avec la première combinaison de matériaux est effectué au moyen d'un premier processeur et le rendu (S103) du deuxième modèle dentaire numérique (200-2) avec la deuxième combinaison de matériaux est effectué au moyen d'un deuxième processeur.

4. Procédé selon l'une des revendications précédentes, où le premier et/ou le deuxième écart ($\Delta E1_{S,I}$, $\Delta E2_{S,I}$) est calculé sur la base d'une distance euclidienne entre le jeu de données cible (DS-S) et le jeu de données réel (DS-I) ou sur la base d'une distance spectrale entre le jeu de données cible (DS-S) et le jeu de données réel (DS-I).

5. Procédé selon l'une des revendications précédentes, où la première et/ou la deuxième combinaison de matériaux comprend au moins deux matériaux de restauration différents (201-1, ..., 203-1, ..., 203-1, ..., 203-3) .

6. Procédé selon l'une des revendications précédentes, où la structure spatiale du premier et/ou du deuxième modèle dentaire numérique (200-1, 200-2) est prédéterminée.

7. Procédé selon l'une des revendications précédentes, où le rendu (S101, S103) est effectué sur la base de valeurs de couleur, de valeurs de réflexion, de valeurs de transmission et/ou d'absorption des matériaux de restauration respectifs.

8. Procédé selon l'une des revendications précédentes, où le rendu (S101, S103) tient compte en outre, lors du rendu, des propriétés optiques d'une couche de ciment, d'une couche de composite, d'une couche d'adhésif et/ou de l'impression de couleur de la préparation.

9. Procédé selon l'une des revendications précédentes, où un nombre déterminé de matériaux de restauration est prédéfini et le procédé est répété pour toutes les combinaisons de matériaux possibles.

10. Procédé selon la revendication 9, où la combinaison de matériaux pour la fabrication qui présente le plus faible écart entre l'ensemble de données de consigne (DS-S) et l'ensemble de données réel respectif est sélectionnée.

11. Procédé selon l'une des revendications précédentes, où le modèle dentaire numérique présente une forme extérieure prédéterminée et une structure interne prédéterminée.

12. Procédé selon l'une des revendications précédentes, où le jeu de données de consigne (DS-S) est obtenu sur la base d'une dent naturelle.

13. Procédé selon la revendication 12, où l'ensemble de données de consigne (DS-S) reproduit les propriétés optiques et/ou la géométrie de la dent naturelle.

14. Dispositif informatique de fabrication d'une restauration dentaire (100), comprenant un dispositif de fabrication apte à mettre en œuvre le procédé selon l'une des revendications 1 à 13.

15. Programme informatique comprenant des instructions pour que le dispositif informatique de la revendication 14 exécute les étapes du procédé selon l'une des revendications 1 à 13.

Fig. 1

Fig. 2

DS-S

$\Delta E_{S, I}$

DS-I

101

Rendering

200

Fig. 3

EP 3 984 493 B1

Fig. 4

EP 3 984 493 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019023461 A1